# EUROPEAN PATENT SPECIFICATION

(11) **EP 1 513 588 B1**
(45) Date of publication and mention of the grant of the patent: **07.05.2008**
(21) Application number: 03755980.4
(22) Date of filing: 22.05.2003
(51) Int. Cl.: A61P 15/00, A61K 31/569, A61K 31/565

(54) **USE OF NEW ETONOGESTREL ESTERS**
VERWENDUNG NEUER ETONOGESTRELESTER
UTILISATION DE NOUVEAUX ESTERS D'ETONOGESTREL

(30) Priority: 30.05.2002 EP 02077118
(43) Date of publication of application: 16.03.2005
(73) Proprietor: N.V. Organon, 5349 AB Oss (NL)
(72) Inventor: De Nijs, Henrik, 5340 BH Oss (NL); Van Der Voort, Hendrikus Adrianus Antonius, 5340 BH Oss (NL); Leysen, Dirk, 5340 BH Oss (NL); Grootenhuis, Arij Jan, 5340 BH Oss (NL); Van Der Louw, Jaap, 5340 BH Oss (NL)
(74) Representative: Kraak, Hajo
(86) International application number: PCT/EP2003/050188
(87) International publication number: WO 2003/101374

(56) References cited:
- EP-A- 0 737 477
- WO-A-00/42942
- WO-A-94/04157
- WO-A-97/03709
- WO-A-99/67270
- WO-A-99/67271
- DE-A- 4 240 806
- DATABASE WPI Week 199524 Derwent Publications Ltd., London, GB; AN 1995-182895 XP002265978 & JP 07 101884 A (SEKISUI CHEM IND CO LTD) , 18 April 1995 (1995-04-18)

## Description

### FIELD OF THE INVENTION

The subject invention concerns the field of (male and female) contraception, (male and female) and hormone replacement therapy (HRT).

### BACKGROUND

Contraceptive methods for men and women are important for worldwide reproductive health.

However, no effective and efficient methods of male contraception are as of yet available.

Male contraception seeks to suppress spermatogenesis through the suppression of the gonadotropins luteinizing hormone (LH) and follicle-stimulating hormone (FSH). This results in a depletion of intratesticular testosterone and cessation of spermatogenesis.

Administration of progestagen results in a dose dependent suppression of pituitary gonadotrophins and consequently, a decrease in testosterone levels and a reversible inhibition of spermatogenesis. An exogenous androgen is required to compensate for the reduced testosterone levels. In the same way, male HRT can be accomplished, resulting in replacement of testosterone by an exogenous androgen which is safer on the prostate than endogenous testosterone.

The use of progestogens together with androgens for use as male contraceptives and HRT is known (Guerin and Rollet (1988), International Journal of Andrology 11, 187-199).

However, the use of specific esters of etonogestrel for male contraception and HRT has not been suggested.

In addition, the use of progestogens together with estrogens for use in female contraception and HRT is known (M. Tausk, J.H.H. Thijssen, Tj.B. van Wimersma Greidanus, "Pharmakologie der Hormone", Georg Thieme Verlag, Stuttgart, 1986).

Progestagens are widely used for female contraception and in female HRT. In contraception, the combination progestagen-estrogen oral contraceptives are the most widely used. Administration of such a combination results in a number of effects: it blocks ovulation, it interferes with phasic development of the endometrium which decreases the chance for successful implantation, and it causes the cervical mucus to become so viscous that it hinders sperm penetration. Most progestagen-only-pills (POP's) aim at the last mentioned effect only.

Female HRT is aimed at suppletion of endogenous estrogen for the treatment of peri- and postmenopausal complaints (hot flushes, vaginal dryness), and for prevention of symptoms of long-term estrogen deficiency. The latter include osteoporosis, coronary artery disease, urogenital incontinence, and possibly also Alzheimer's disease and colorectal cancer. A drawback of long-term unopposed estrogen administration is the associated increase in endometrium proliferation, which in turn may increase the risk of endometrial cancer. For that reason, progestagens are co-administered in long-term regimes, because of their ability to reduce the proliferative activity of endometrial epithelium and to induce secretory conversion.

However, the use of specific C10-C12 esters of etonogestrel for female contraception, and female HRT has not been suggested; WO 97/03709 focuses on etonogestrel esters with a fatty chain length of C2, C4 and C6. The subject invention describes esters of etonogestrel with a fatty-chain length of C10-C12, which have a good pharmacokinetic profile and enable a single-dose administration of a progestogen with a long duration of action.

The subject invention provides a male and female contraceptive and/or HRT kit comprising a contraceptively and/or therapeutically effective amount of a long-acting etonogestrel ester with a fatty-chain length of C10-C12.

### FIGURES

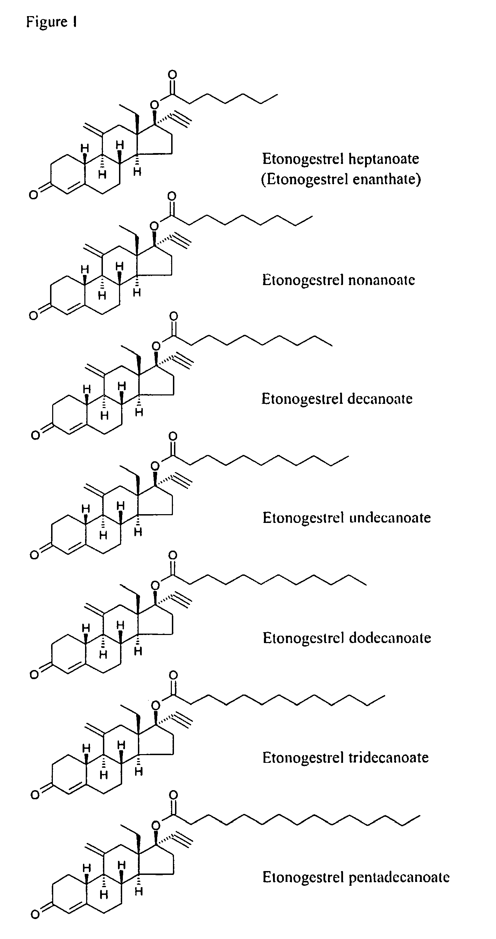

In the figures, any references to esters having chain lengths other than C10, C11 or C12 do not form part of the invention as claimed. Figure 1

Chemical structures of etonogestrel heptanoate (etonogestrel enanthate), etonogestrel nonanoate, etonogestrel decanoate, etonogestrel undecanoate, etonogestrel dodecanoate, etonogestrel tridecanoate, and etonogestrel pentadecanoate.

### Figure 2a

Effect of one intramuscular (IM) injection of etonogestrel, etonogestrel heptanoate (etonogestrel enanthate), etonogestrel nonanoate and etonogestrel undecanoate on plasma levels of etonogestrel in male intact rabbits. Means and SEM of N=3.

### Figure 2b

Effect of one intramuscular (IM) injection of etonogestrel heptanoate (etonogestrel enanthate), etonogestrel nonanoate, etonogestrel decanoate, etonogestrel undecanoate, etonogestrel dodecanoate, etonogestrel tridecanoate on plasma levels of etonogestrel in male intact rabbits. Means and SEM of N=3.

### Figure 3

Chemical structure of MENT-undecanoate, MENT-buciclate, testosterone heptanoate (testosterone enanthate) and testosterone undecanoate.

### Figure 4

Time dependent effects of one s.c injection of 20 mg/kg of MENT-undecanoate (MENT-U), MENT-buciclate (MENT-B), testosterone-heptanoate (testosterone-enanthate, TE) and testosterone-undecanoate (TU) in castrated male rabbits on serum MENT or testosterone (T). Results are means of N=3.

### Figure 5

Pharmacokinetics of testosterone enanthate, testosterone undecanoate and testosterone buciclate after one IM injected in male hypogonadal men with indicated doses on the plasma levels of serum testosterone. Normal range of serum testosterone is indicated with a dashed line. Derived from E. Nieschlag and H.M. Behre. Testosterone Therapy. In: Andrology, Male reproductive health and dysfunction., edited by E. Nieschlag and H. M. Behre, Berlin, Heidelberg and New York:Springer-Verlag, 1997, p. 297-309.

### DETAILED DESCRIPTION OF THE INVENTION

The subject invention provides a contraceptive and/or HRT kit comprising a contraceptively and/or therapeutically effective amount of a long-acting etonogestrel ester with a fatty-chain length of C10-C12.

In one embodiment, the kit further comprises a contraceptively and/or therapeutically effective amount of an androgen ester, preferably MENT undecanoate.

In another embodiment, the kit further comprises a contraceptively and/or therapeutically effective amount of an estrogen, such as mestranol, ethynylestradiol, ethynylestradiol sulfonate, estradiol, estradiol valerate, estriol, estriol succinate, quinestrol, estropipate, sodium estrone sulfate or sodium equilin sulfate.

The subject invention further contemplates a use of a contraceptively and/or therapeutically effective amount of a long-acting etonogestrel ester with a fatty-chain length of C10-C12, for the preparation of a medicament for contraception and/or HRT.

In one embodiment, the etonogestrel ester is used in conjunction with a contraceptively and/or therapeutically effective amount of a long-acting androgen ester for the preparation of a medicament for male contraception and/or male HRT. The long-acting androgen ester is preferably MENT undecanoate.

In another embodiment, the etonogestrel ester is used in conjunction with a contraceptively and/or therapeutically effective amount of an estrogen for the preparation of a medicament for female contraception and/or female HRT.

The subject invention also encompasses treatments contraception and/or HRT comprising administering to a subject a contraceptively and/or therapeutically effective amount of a long-acting etonogestrel ester with a fatty-chain length of C10-C12.

In one embodiment, a contraceptively and/or therapeutically effective amount of a long-acting androgen ester, preferably MENT undecanoate, is administered in conjunction with the etonogestrel ester.

In another embodiment, a contraceptively and/or therapeutically effective amount of an estrogen is administered in conjunction with the etonogestrel ester.

In the above aspects of the subject invention, the contraceptively and/or therapeutically effective amount of MENT undecanoate is 50-400 mg and the contraceptively and/or therapeutically effective amount of etonogestrel ester is 25-200 mg.

In a preferred embodiment, the contraceptively and/or therapeutically effective amount of MENT undecanoate is 50-200 mg and the contraceptively and/or therapeutically effective amount of etonogestrel ester is 50-100 mg.

In a specifically preferred embodiment, the contraceptively and/or therapeutically effective amount of MENT undecanoate is 100 mg and the contraceptively and/or therapeutically effective amount of etonogestrel ester is 50 mg.

The progestogen and testosterone esters can be prepared by dissolving it in a suitable amount of an oily medium, such as arachis oil, oleic acid, castor oil, ethyl undecanoate, almond oil, sesame oil, coconut oil, olive oil, soyabean oil, (purified) tri-glycerised, propylene glycol esters, ethyl oleate and the like, including mixtures of oils. The amount of esters that can be dissolved differs per chosen medium, but will generally be within the range of from 100-400 mg. The preferred oil is arachis oil or ethyl undecanoate.

Additives common to injection fluids can be added to the solution if desired. Suitable additives are known to the person skilled in the art. Possible additives include liquids that serve to lower the viscosity of the formulation, e.g. benzyl alcohol, benzyl benzoate, benzyl propionate, ethyl oleate or ethyl undecanoate.

The compounds of the subject invention can principally be administered via any suitable route available to the skilled person.

In the case of oral administration, a solid dosage unit such as a tablet or a capsule is contemplated. The compounds of the invention can be formulated with a pharmaceutically acceptable carrier, such as described in the standard reference, Gennaro et al, Remmington: The Science and Practice of Pharmacy, (20th ed., Lippincott Williams & Wilkins, 2000, see especially Part 5: Pharmaceutical Manufacturing). The compounds of the invention and the pharmaceutically acceptable carrier may be compressed into solid dosage units, such as pills, tablets, or be processed into capsules or suppositories. By means of pharmaceutically suitable liquids the compounds can also be applied as an injection preparation in the form of a solution, suspension, emulsion, or as a spray, *e*.*g*. nasal spray. For making dosage units, *e*.*g*. tablets, the use of conventional additives such as fillers, colorants, polymeric binders, lubricants, flow enhancers, glidants and the like is contemplated. In general any pharmaceutically acceptable additive which does not interfere with the function of the active compounds can be used. The compounds of the invention may also be included in an implant, a vaginal ring, a patch, a gel, and the like.

Suitable carriers with which the compositions can be administered include lactose, starch, cellulose derivatives and the like, or mixtures thereof used in suitable amounts.

The dose of and regimen of administration of the compounds of the invention, or a pharmaceutical composition thereof, to be administered will depend on the therapeutic effect to be achieved and will vary with the route of administration, and the age and condition of the individual subject to whom the medicament is to be administered, and/or the particular contraceptive or HRT regimen in which it is used. Typical dosage amounts are 0.001-5 mg per kg body weight.

The present invention is further described in the following examples.

### EXAMPLES

Any reference in the examples to esters having chain lengths other than C10, C11 or C12 does not form part of the invention as claimed.

### EXAMPLE 1-Kinetics of etonogestrel C7, C9, C10, C11, C12 and C13 esters in rabbits

The following etonogestrel esters were prepared and tested in rabbits:
● Etonogestrel heptanoate
● Etonogestrel nonanoate
● Etonogestrel decanoate
● Etonogestrel undecanoate
● Etonogestrel dodecanoate
● Etonogestrel tridecanoate

Etonogestrel pentadecanoate was also prepared.

Figure 1 shows the chemical structure of these compounds.

As a reference, etonogestrel was also included.

### Preparation of etonogestrel esters

General methodology for the preparation of esters from alcohols can be found in e.g. Greene, T.W. et al, "Protective groups in organic synthesis", John Wiley & Sons, NY, 1999 (third edition). Preparation of esters from tertiary alcohols (like etonogestrel) can be accomplished by several techniques, for instance:
1) tertiary alcohol, carboxylic acid, trifluoroacetic acid-anhydride, DE 1013284 (1956); 2) tertiary alcohol, acid chloride, pyridine, Watson, T.G. et al, Steroids 41, 255 (1983); 3) tertiary alcohol, acid chloride, TlOEt, Shafiee, A. et al, Steroids 41, 349 (1983), 4) tertiary alcohol, carboxylic acid-anhydride, TsOH, benzene, Johnson, A.L., Steroids, 20, 263 (1972); and 5) tertiary alcohol, carboxylic acid-anhydride, DMAP, CH₂Cl₂, Shafiee, A. et al, Steroids 41, 349 (1983).

### Preparation of (17α)-13-Ethyl-11-methylene-17-[[(1-oxononyl)oxy]-18,19-dinorpregn-4-en-20-yn-3-one (etonogestrel nonanoate)

a) A solution of nonanoic acid (1.95 g) in dry toluene (8 ml) was cooled to 0 °C and treated with trifluoroacetic acid anhydride (2.6 g). After 30 min. stirring, (17α)-13-ethyl-17-hydroxy-11-methylene-18,19-dinorpregn-4-en-20-yn-3-one (etonogestrel, 2.0 g) in dry toluene (15 ml) was added and the reaction mixture was stirred for 17 h at room temperature. The reaction mixture was washed with water, a saturated aqueous solution of sodium hydrogen carbonate, water, and brine. The organic phase was dried over sodium sulfate and concentrated under reduced pressure. The residue was purified by column chromatography (toluene/ethyl acetate 95:5). The product (2.08 g) was dissolved in ethyl acetate (40 ml), cooled to 0 °C, and stirred with aqueous sodium hydroxide (1 M, 13 ml) for 2 h. The mixture was extracted with ethyl acetate; the combined organic phases were washed with ice-cold aqueous sodium hydroxide (1 M), water and brine, dried and concentrated under reduce pressure. Column chromatography afforded (17α)-13-ethyl-11-methylene-17-[[(1-oxononyl)oxy]-18,19-dinorpregn-4-en-20-yn-3-one (1.25 g). ¹H-NMR (CDCI₃): δ 5.89 (m, I H), 5.08 (bs, I H), 4.85 (bs, 1H), 2.82 (ddd, 1H, J = 14.8, 9.5 and 6.3 Hz), 2.73 (d, 1H, J = 12.8 Hz), 2.69-2.19 (m), 2.63 (s, 1H), 2.11 (m, 1H), 1.90-1.21 (m), 1.15 (m, 1H), 1.05 (t, 3H, J = 7.5 Hz), 0.88 (t, 3H, J = 7.1 Hz). Measured mass [M+H]⁺ 465.3358. Calculated mass [M+H]⁺ 465.3363.
   In a manner analogous to the procedure described above, etonogestrel heptanoate, etonogestrel decanoate, etonogestrel undecanoate, etonogestrel dodecanoate, etonogestrel tridecanoate, and etonogestrel pentadecanoate were prepared:
b) (17α)-13-Ethyl-11-methylene-17-[[(1-oxoheptyl)oxy]-18,19-dinorpregn-4-en-20-yn-3-one (etonogestrel heptanoate). ¹H-NMR (CDCI₃): δ 5.89 (m, 1H), 5.08 (bs, 1H), 4.85 (bs, 1H), 2.82 (ddd, 1H, J = 14.8, 9.5 and 6.3 Hz), 2.73 (d, 1H, J = 12.6 Hz), 2.68-2.19 (m), 2.63 (s, I H), 2.11 (m, I H), 1.90-1.24 (m), 1.15 (m, I H), 1.05 (t, 3H, J = 7.5 Hz), 0.89 (t, 3H, J = 7.1 Hz). Measured mass [M+H]⁺ 437.3027. Calculated mass [M+H]⁺ 437.3050.
c) (17α)-13-Ethyl-11-methylene-17-[[(1-oxodecyl)oxy]-18,19-dinorpregn-4-en-20-yn-3-one (etonogestrel decanoate). ¹H-NMR (CDCI₃): δ 5.89 (bs, 1H), 5.08 (bs, I H), 4.84 (bs, 1H), 2.82 (m, I H), 2.73 (d, 1H, J = 12.6 Hz), 2.67-2.18 (m), 2.63 (s, 1H), 2.11 (m, 1H), 1.90-1.21 (m), 1.15 (m, I H), 1.06 (t, 3H, J = 7.5 Hz), 0.88 (t, 3H, J = 7.1 Hz). Measured mass [M+H]⁺ 479.3508. Calculated mass [M+H]⁺ 479.3519.
d) (17α)-13-Ethyl-11-methylene-17-[[(1-oxoundecyl)oxy]-18,19-dinorpregn-4-en-20-yn-3-one (etonogestrel undecanoate). ¹H-NMR (CDCl₃): δ 5.89 (m, 1H), 5.08 (bs, 1H), 4.85 (bs, 1H), 2.82 (ddd, 1H, J = 14.8, 9.5 and 6.3 Hz), 2.73 (d, 1H, J = 12.6 Hz), 2.68-2.18 (m), 2.63 (s, I H), 2.11 (m, 1H), 1.90-1.21 (m), 1.06 ( t, 3H, J = 7.5 Hz), 0.88 (t, 3H, J = 7.1 Hz). Measured mass [M+H]⁺ 493.3664. Calculated mass [M+H]⁺ 493.3676.
e) (17α)-13-Ethyl-11-methylene-17-[[(1-oxododecyl)oxy]-18,19-dinorpregn-4-en-20-yn-3-one (etonogestrel dodecanoate). ¹H-NMR (CDCI₃): δ 5.89 (bs, I H), 5.08 (bs, 1H), 4.85 (bs, 1H), 2.82 (m, 1H), 2.73 (d, 1H, J = 12.6 Hz), 2.65-2.18 (m), 2.64 (s, 1H), 2.11 (m, I H), 1.90-1.20 (m), 1.15 (m, I H), 1.06 (t, 3H, J = 7.5 Hz), 0.88 (t, 3H, J = 7.1 Hz). Measured mass [M+H]⁺ 507.3829. Calculated mass [M+H]⁺ 507.3832.
f) (17α)-13-Ethyl-11-methylene-17-[[(1-oxotridecyl)oxy]-18,19-dinorpregn-4-en-20-yn-3-one (etonogestrel tridecanoate). ¹H-NMR (CDCl₃): δ 5.89 (bs, 1H), 5.08 (bs, 1H), 4.85 (bs, 1H), 2.82 (m, 1H), 2.73 (d, 1H, J = 12.6 Hz), 2.65-2.18 (m), 2.64 (s, 1H), 2.11 (m, 1H), 1.90-1.20 (m), 1.15 (m, 1H), 1.06 (t, 3H, J = 7.5 Hz), 0.89 (t, 3H, J = 7.1 Hz). Measured mass [M+H]⁺ 521.4007. Calculated mass [M+H]⁺ 521.3989.
g) (17α)-13-Ethyl-11-methylene-17-[[(1-oxopentadecyl)oxy]-18,19-dinorpregn-4-en-20-yn-3-one (etonogestrel pentadecanoate). ¹H-NMR (CDCl₃): δ 5.89 (bs, 1H), 5.08 (bs, I H), 4.85 (bs, 1H), 2.82 (m, 1H), 2.73 (d, 1H, J = 12.6 Hz), 2.65-2.19 (m), 2.63 (s, 1H), 2.11 (m, 1H), 1.90-1.20 (m), 1.15 (m, 1H), 1.06 (t, 3 H, J = 7.5 Hz), 0.89 (t, 3H, J = 7.1 Hz). Measured mass [M+H]⁺ 549.4278. Calculated mass [M+H]⁺ 549.4302.

### Pharmacokinetics studies in the rabbit

For the determination of the pharmacokinetic profile of the different etonogestrel-esters after parenteral application, i.m. application in the castrated rabbit model was chosen instead of s.c. Briefly, rabbits were injected once (day 1) with indicated etonogestrel-esters at 20 mg/kg in arachis oil (with a concentration of 40 mg/ml). At day 1, 2, 3, 4, 5, 6, 7, 8, 10, 12, 14, 21, 28, 35, 49, 63, 77, 92, 106, 120 and 133 blood was collected from the ear arteria, in EDTA-containing tubes. EDTA plasma was prepared (1500g, 15 min) and stored at -20°C. With LC- MSMS the amount of parent compound (etonogestrel) was determined in these samples. The lower limit ofthis new assay is 0.5 nmol/l, from 0-250 nmol/l a linear curve was obtained with a correlation coefficient of 0,9998.

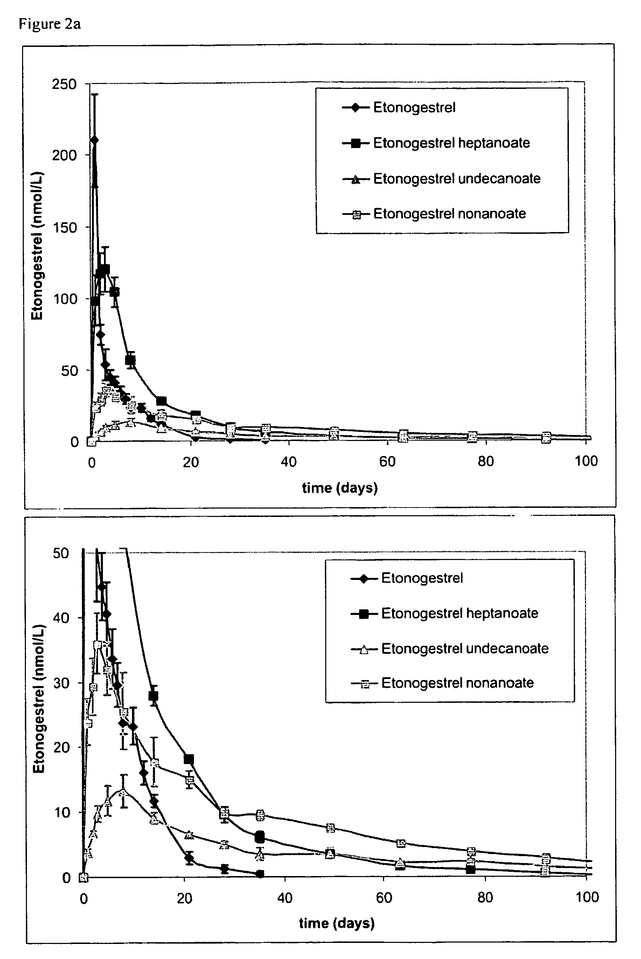

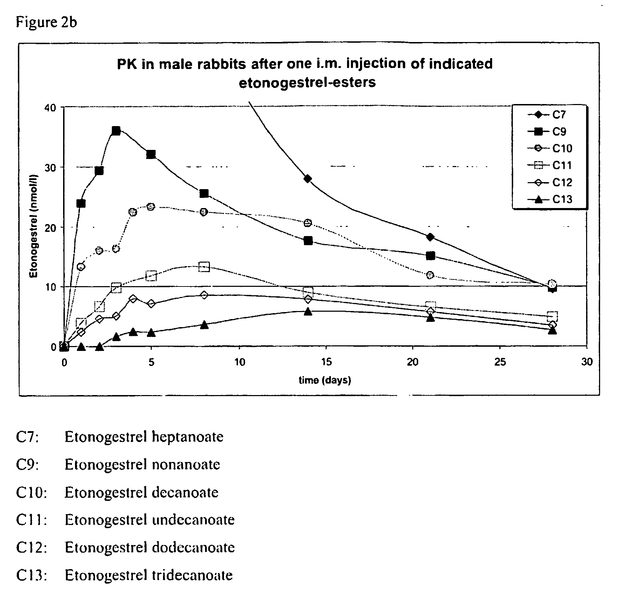

As shown in Figure 2a, etonogestrel itself resulted in very high peak levels (200 nmol/l), which declined in 28 days to levels of etonogestrel below 1 nmol/l. Etonogestrel-heptanoate also gave rise to high initial peak levels of etonogestrel (120 nmol/l). Etonogestrel nonanoate gave lower peak levels and extended duration with serum levels of etonogestrel above 1 nmol/l. As compared to the other two esters in Figure 2a, etonogestrel undecanoate gave the most optimal balance between initial peak levels (maximum of 13 nmol/l after eight days) and duration of action (more than 92 days above 1 nmol/l).
As shown in Figure 2b, etonogestrel decanoate gave an initial peak level of 24 nmol/l after 5 days whereas etonogestrel dodecanoate gave an initial peak level of 9 nmol/l after 8 days. With etonogestrel tridecanoate, no initial levels of etonogestrel were observed.
From Figures 2a and 2b, it can be seen that preferred etonogestrel esters are etonogestrel decanoate, etonogestrel undecanoate, and etonogestrel dodecanoate.

### EXAMPLE 2-Kinetics of two MENT esters in rabbits

The pharmacokinetic profile of MENT-undecanoate and MENT-buciclate was compared to testosterone-enanthate and testosterone-undecanoate.

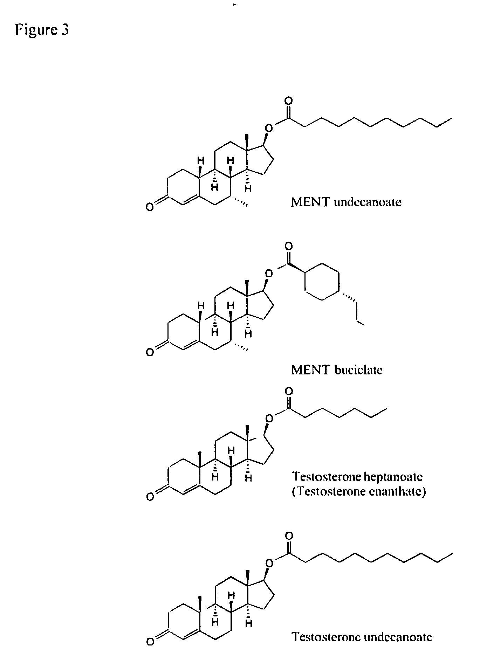

Figure 3 shows the chemical structures of these androgen esters.

Ment-undecanoate was prepared essentially as described in WO 99/67271. MENT-buciclate was prepared as described in WO 99/67270. Testosterone enanthate and undecanoate were commercially obtained from Diosynth, Oss, the Netherlands.

### Pharmacokinetic studies in the rabbit

For the determination of the pharmacokinetic profile of the different androgen-esters after s.c. application, the castrated rabbit model was selected as the model which is most similar to humans. Briefly, rabbits were injected once (day 1) with indicated androgen-esters at 20 mg/kg in arachis oil (with a concentration of 100 mg/ml). At day 2, 3, 4, 5, 8, 15, 22, 36, 44 and 58 blood was collected from the ear arteria, in EDTA-containing tubes. EDTA plasma was prepared (1500g, 15 min) and stored at - 20°C. With LC-MSMS, the amount of parent compound (testosterone or MENT) was determined in these samples. The lower limit of this new assay is 2 nmol/l, from 0-500 nmol/l a linear curve was obtained with a correlation coefficient of 0,9998.

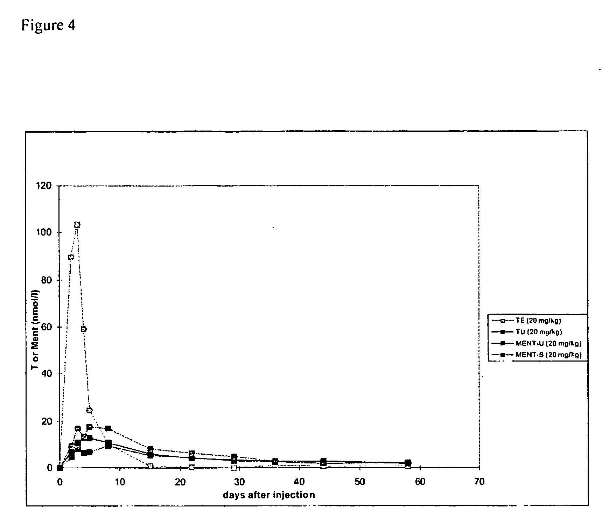

As shown in Figure 4, both with MENT-undecanoate and MENT-buciclate a pharmacokinetic profile of released MENT was found which is similar to that of the reference compound testosterone-undecanoate with respect to released testosterone. Testosterone-enanthate resulted in a high peak of testosterone 2 days after injection.

Thus, in the rabbit, with both MENT-esters no initial rise of MENT was observed on one hand and a prolonged release of MENT was observed on the other hand, suggestive for more optimal pharmacokinetic behaviour than the current standard testosterone-enanthate.

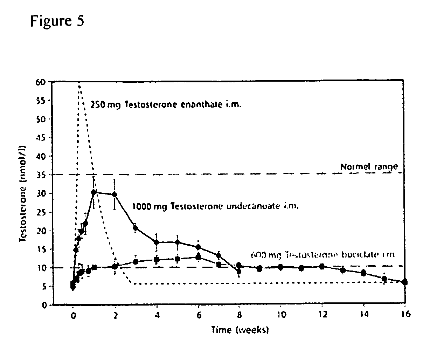

In humans, optimal pharmacokinetics were obtained with testosterone undecanoate: low initial release and steady-state levels of long duration (Figure 5). Since in rabbits the pharmacokinetic profile of the two MENT-esters was very similar to that of testosterone-undecanoate (Figure 4), optimal pharmacokinetics with both MENT esters in humans is expected.

### EXAMPLE 3- Solubility and viscosity of MENT-undecanoate and etonogestrel undecanoate in various solvents

To determine the solubility and viscosity of MENT undecanoate and etonogestrel undecanoate, four different solvents were used:
● ethyl undecanoate
● ethyl undecanoate + 50% benzyl benzoate
● arachis oil
● arachis oil + 50% benzyl benzoate

Using these solvents, the following solutions were prepared:
● 100 mg/ml etonogestrel undecanoate in the different solvents
● 50 mg/ml etonogestrel undecanoate in the different solvents
● 200 mg/ml MENT undecanoate in the different solvents
● 100 mg/ml MENT undecanoate in the different solvents
● 50 mg/ml etonogestrel undecanoate + 100 mg/ml MENT undecanoate in the different solvents

The two combined solvents were prepared by addition of 50 gram of ethyl undecanoate or arachis oil to 50 gram of benzyl benzoate. The ethyl undecanoate + 50% benzyl benzoate solution was filtered over a 0.22 µm Durapore filter to obtain a clear colourless solution. The arachis oil + 50% benzyl benzoate solution was not filtered.

The solubility of the compounds in the solvents was determined visually. The viscosity was determined using a Brookfield model DV-III. The density of the solutions was determined using a Mettler Toledo DA-100M density meter.

**Table 1 : Appearance, viscosity and density of the solvents**

| **Solvent** | **Appearance** | **Viscosity** | **Density** |
|---|---|---|---|
| Ethyl undecanoate | Clear colourless | 2.6 | 0.861 |
| | solution | | |
| Ethyl undecanoate + | Clear colourless | 3.9 | 0.975 |
| 50% benzyl benzoate | solution | | |
| Arachis oil | Clear yellowish | 64.1 | 0.913 |
| | solution | | |
| Arachis oil + 50% | Clear yellowish | 22.9 | 1.007 |
| benzyl benzoate | solution | | |
| Benzyl benzoate | Clear yellowish | 8.5 | 1.117 |
| | solution | | |

Ethyl undecanoate, ethyl undecanoate + 50% benzyl benzoate and arachis oil + 50% benzyl benzoate solutions did not need to be heated. To dissolve 200 mg/ml MENT undecanoate in arachis oil, heating to approximately 50°C was necessary.

The concentrations tested were 100 mg/ml etonogestrel undecanoate, 200 mg/ml MENT undecanoate and 50 mg/ml etonogestrel undecanoate + 100 mg/ml MENT undecanoate in the different solvents. The results are summarized in table 2.

**Table 2: Appearance, viscosity and density of the final solutions**

| **Solvent** | **Etonogestrel** | **MENT** | **Appearance** | **Viscosity** | **Density** |
|---|---|---|---|---|---|
| | **undecanoate** | **undecanoate** | | **(cps)** | **(g/ml**) |
| | **(mg/ml)** | **(mg/ml)** | | | |
| Ethyl undecanoate | 50 | - | Clear colourless | 3.2 | 0.870 |
| | - | 100 | solution | 4.0 | 0.879 |
| | 50 | 100 | Clear colourless | 4.4 | 0.886 |
| | | | solution | | |
| | | | Clear colourless | | |
| | | | solution | | |
| Ethyl undecanoate | 50 | - | Clear colourless | 4.7 | 0.978 |
| + 50% benzyl | - | 100 | solution | 6.1 | 0.979 |
| benzoate | 50 | 100 | Clear colourless | 7.0 | 0.979 |
| | | | solution | | |
| | | | Clear colourless | | |
| | | | solution | | |
| Arachis oil | 50 | - | Clear yellowish | 76.6 | 0.919 |
| | - | 100 | solution | 97.2 | 0.924 |
| | 50 | 100 | Clear yellowish | 99.7 | 0.935 |
| | | | solution | | |
| | | | Clear yellowish | | |
| | | | solution | | |
| Arachis oil + 50% | 50 | | Clear yellowish | 28.1 | 1.006 |
| benzyl benzoate | - | 100 | solution | 35.0 | 1.009 |
| | 50 | 100 | Clear yellowish | 39.1 | 1.008 |
| | | | solution | | |
| | | | Clear yellowish | | |
| | | | solution | | |

The combination of etonogestrel-undecanoate and MENT-undecanoate was visually dissolved at a desired concentration of 50 mg/ml etonogestrel-undecanoate and 100 mg/ml MENT-undecanoate in all four tested solvents. Both etonogestrel-undecanoate and MENT-undecanoate could be dissolved at two times the desired concentration in all four solvents tested. No precipitation occurred at room temperature when 50 mg/ml etonogestrel-undecanoate and 100 mg/ml MENT-undecanoate were dissolved in all four solvents.

The viscosity of ethyl undecanoate and ethyl undecanoate + 50% benzyl benzoate was significantly lower than the viscosity of arachis oil and arachis oil + 50% benzyl benzoate. The viscosity of the desired formulation 50 mg/ml etonogestrel undecanoate + 100 mg/ml MENT undecanoate in the four different solvents was the lowest ( 4 cps) for the ethyl undecanoate solution, followed by the ethyl undecanoate + 50% benzyl benzoate (7 cps) and the arachis oil + 50% benzyl benzoate solution (39 cps). The viscosity of the arachis oil solution was significantly higher that the viscosity of the other solutions (100 cps).

### EXAMPLE 4 - Pharmacological action of etonogestrel esters in the male

The pharmacological action of etonogestrel esters in the male are evaluated for the suppressing activity of endogenous testosterone in the rabbit as described in Wu,F.C., Balasubramanian,R., Mulders,T. M. and Coelingh-Bennink H.J., Oral progestogen combined with testosterone as a potential male contraceptive: additive effects between desogestrel and testosterone enanthate in suppression of spermatogenesis, pituitary-testicular axis, and lipid metabolism, J.Clin.Endocrinol.Metab 84 (1):112-122, 1999. Briefly, the effect of one sc/im injection of the different etonogestrel esters on serum testosterone at day 7 of mature male rabbits will be monitored.

### EXAMPLE 5 - The pharmacological action of etonogestrel esters in the female

The pharmacological action of etonogestrel esters in the female are tested in the classical Clauberg test. Briefly, immature female rabbits, primed with oestradiol for 8 days, are treated once sc/im with the different etonogestrel esters (day 8 afternoon). Autopsy is performed in the afternoon of day 13 and the progestagenic activity is evaluated on sections of the uterine according to McPhail et al., The assay of progestin. J. of Physiology, 1934, 83:145-156*.*

## Claims

1. An oily pharmaceutical composition for use as contraceptive and/or HRT comprising a contraceptively and/or therapeutically effective amount of etonogestrel undecanoate and/or etonogestrel decanoate and/or etonogestrel dodecanoate.

2. The pharmaceutical composition according to claim 1 further comprising a contraceptively and/or therapeutically effective amount of an androgen ester.

3. The pharmaceutical composition according to claim 2 wherein the androgen ester is MENT undecanoate.

4. The pharmaceutical composition according to claim 3 wherein the amount of MENT undecanoate is 50-400 mg and the amount of etonogestrel undecanoate and/or etonogestrel decanoate and/or etonogestrel dodecanoate is 25-200 mg.

5. The pharmaceutical composition according to claim 1 further comprising a contraceptively and/or therapeutically effective amount of an estrogen.

6. The use of etonogestrel undecanoate or etonogestrel decanoate or etonogestrel dodecanoate for the preparation of a medicament for contraception and/or HRT whereby the treatment is by the injection route of administration of etonogestrel undecanoate and/or etonogestrel decanoate and/or etonogestrel dodecanoate in an oily medium.

7. The use of etonogestrel undecanoate or etonogestrel decanoate or etonogestrel dodecanoate for the preparation of a medicament for male contraception and/or male HRT whereby the medicament also comprises a contraceptively and/or therapeutically effective amount of a long-acting androgen ester.

8. The use according to claim 7, wherein the contraceptively and/or therapeutically effective and long-acting androgen ester is MENT undecanoate.

9. The use according to claim 8, wherein the contraceptively and/or therapeutically effective amount of MENT undecanoate is 50-400 mg and the contraceptively and/or therapeutically effective amount of etonogestrel undecanoate and/or etonogestrel decanoate and/or etonogestrel dodecanoate is 25-200 mg.

10. The use of etonogestrel undecanoate or etonogestrel decanoate for the preparation of a medicament for female contraception and/or female HRT, whereby the medicament also comprises a contraceptively and/or therapeutically effective amount of an estrogen.

11. The use of etonogestrel undecanoate or etonogestrel decanoate or etonogestrel dodecanoate for the preparation of a medicament for female contraception and/or female HRT, whereby the medicament is for the injection route of administration of etonogestrel undecanoate and/or etonogestrel decanoate and/or etonogestrel dodecanoate in an oily medium.

## Patentansprüche

1. Ölige pharmazeutische Zusammensetzung für die Verwendung als Verhütungsmittel und/oder in der Hormonersatztherapie, umfassend ein als Verhütungsmittel und/oder therapeutisch wirksame Menge eines Etonogestrel-Undecanoats und/oder Etonogestrel-Decanoats und/oder Etonogestrel-Dodecanoats.

2. Pharmazeutische Zusammensetzung nach Anspruch 1, weiterhin umfassend eine als Verhütungsmittel und/oder therapeutisch wirksame Menge eines Androgenesters.

3. Pharmazeutische Zusammensetzung nach Anspruch 2, bei der der Androgenester MENT-Undecanoat ist.

4. Pharmazeutische Zusammensetzung nach Anspruch 3, bei der die Menge an MENT-Undecanoat zwischen 50 und 400 mg und die Menge an Etonogestrel-Undecanoat und/oder Etonogestrel-Deconaot und/oder Etonogestrel-Dodecanoat zwischen 25 und 200 mg liegt.

5. Pharmazeutische Zusammensetzung nach Anspruch 1, weiterhin umfassend ein als Verhütungsmittel und/oder therapeutisch wirksame Menge eines Östrogens.

6. Verwendung von Etonogestrel-Undecanoat oder Etonogestrel-Decanoat oder Etonogestrel-Dodecanoat für die Herstellung eines Medikaments für die Verhütung und/oder Hormonersatz-Therapie, wobei die Behandlung über den Injektionsweg der Verabreichung von Etonogestrel-Undecanoat und/oder Etonogestrel-Decanoat und/oder Etonogestrel-Dodecanoat in einem öligen Medium durchgeführt ist.

7. Verwendung von Etonogestrel-Undecanoat oder Etonogestrel-Decanoat oder Etonogestrel-Dodecanoat für die Herstellung eines Medikaments für die männliche Verhütung und/oder männliche Hormonersatz-Therapie, wobei das Medikament auch eine als Verhütungsmittel und/oder therapeutisch wirksame Menge eines langwirkenden Androgenesters umfasst.

8. Verwendung nach Anspruch 7, bei dem das als Verhütungsmittel und/oder therapeutisch wirksame und langanhaltende Androgenester MENT-Undecanoat ist.

9. Verwendung nach Anspruch 8, bei dem die als Verhütungsmittel und/oder therapeutisch wirksame Menge an MENT-Undecanoat zwischen 50 und 400 mg liegt und die als Verhütungsmittel und/oder therapeutisch wirksame Menge von Etonogestrel-Undecanoat und/oder Etonogestrel-Decanoat und/oder Etonogestrel-Dodecanoat zwischen 25 und 200 mg liegt.

10. Verwendung des Etonogestrel-Undecanoats oder des Etonogestrel-Decanoats für die Herstellung eines Medikaments für die weibliche Verhütung und/oder weibliche Hormonersatztherapie, wobei das Medikament auch ein als Verhütungsmittel und/oder therapeutisch wirksame Menge eines Östrogens umfasst.

11. Verwendung von Etonogestrel-Undecanoat oder Etonogestrel-Decanoat oder Etonogestrel-Dodecanoat für die Herstellung eines Medikaments für die weibliche Verhütung und/oder weibliche Hormonersatz-Therapie, wobei das Medikament für den Injektionsweg der Verabreichung von Etonogestrel-Undecanoat und/oder Etonogestrel-Decanoat und/oder Etonogestrel-Dodecanoat in einem öligen Medium vorgesehen ist.

## Revendications

1. Une composition pharmaceutique huileuse utile en tant que contraceptif et/ou traitement hormonal substitutif comprenant une quantité efficace du point de vue contraceptif et/ou thérapeutique d'undécanoate d'étonogestrel et/ou de décanoate d'étonogestrel et/ou de dodécanoate d'étonogestrel.

2. La composition pharmaceutique selon la revendication 1 comprenant de plus une quantité efficace du point de vue contraceptif et/ou thérapeutique d'un ester d'un androgène.

3. La composition pharmaceutique selon la revendication 2, dans laquelle l'ester d'un androgène est l'undécanoate de MENT.

4. La composition pharmaceutique selon la revendication 3, dans laquelle la quantité d'undécanoate de MENT est de 50 à 400 mg et la quantité d'undécanoate d'étonogestrel et/ou de décanoate d'étonogestrel et/ou de dodécanoate d'étonogestrel est de 25 à 200 mg.

5. La composition pharmaceutique selon la revendication 1 comprenant de plus une quantité efficace du point de vue contraceptif et/ou thérapeutique d'un oestrogène.

6. L'utilisation d'undécanoate d'étonogestrel ou de décanoate d'étonogestrel ou de dodécanoate d'étonogestrel pour la préparation d'un médicament destiné à la contraception et/ou au traitement hormonal substitutif dans laquelle le traitement utilise le mode d'administration par injection d'undécanoate d'étonogestrel et/ou de décanoate d'étonogestrel et/ou de dodécanoate d'étonogestrel dans un milieu huileux.

7. L'utilisation d'undécanoate d'étonogestrel ou de décanoate d'étonogestrel ou de dodécanoate d'étonogestrel pour la préparation d'un médicament destiné à la contraception masculine et/ou au traitement hormonal substitutif masculin dans laquelle le médicament comprend aussi une quantité efficace du point de vue contraceptif et/ou thérapeutique d'un ester d'un androgène à longue durée d'action.

8. L'utilisation selon la revendication 7, dans laquelle l'ester d'un androgène efficace du point de vue contraceptif et/ou thérapeutique et à longue durée d'action est l'undécanoate de MENT.

9. L'utilisation selon la revendication 8, dans laquelle la quantité efficace du point de vue contraceptif et/ou thérapeutique d'undécanoate de MENT est de 50 à 400 mg et la quantité efficace du point de vue contraceptif et/ou thérapeutique d'undécanoate d'étonogestrel et/ou de décanoate d'étonogestrel et/ou de dodécanoate d'étonogestrel est de 25 à 200 mg.

10. L'utilisation d'undécanoate d'étonogestrel ou de décanoate d'étonogestrel pour la préparation d'un médicament destiné à la contraception féminine et/ou au traitement hormonal substitutif féminin dans laquelle le médicament comprend aussi une quantité efficace du point de vue contraceptif et/ou thérapeutique d'un oestrogène.

11. L'utilisation d'undécanoate d'étonogestrel ou de décanoate d'étonogestrel ou de dodécanoate d'étonogestrel pour la préparation d'un médicament destiné à la contraception féminine et/ou au traitement hormonal substitutif féminin dans laquelle le traitement utilise le mode d'administration par injection d'undécanoate d'étonogestrel et/ou de décanoate d'étonogestrel et/ou de dodécanoate d'étonogestrel dans un milieu huileux
